# EUROPÄISCHE PATENTSCHRIFT

(11) **EP 3 552 666 B1**
(45) Veröffentlichungstag und Bekanntmachung des Hinweises auf die Patenterteilung: **21.10.2020**
(21) Anmeldenummer: 19166454.9
(22) Anmeldetag: 01.04.2019
(51) Int. Cl.: A61Q 1/02, A61Q 1/10, A61K 8/73, A61K 8/26, C09C 1/00

(54) **GLITTER ENTHALTEND CELLULOSEACETAT**
GLITTER CONTAINING CELLULOSE ACETATE
PAILLETTES CONTENANT DE L'ACÉTATE DE CELLULOSE

(30) Priorität: 12.04.2018 DE 102018205562
(43) Veröffentlichungstag der Anmeldung: 16.10.2019
(73) Patentinhaber: Sigmund Lindner GmbH, 95485 Warmensteinach (DE)
(72) Erfinder: PSCHIERER, Erwin, 95506 Kastl (DE)
(74) Vertreter: Isarpatent

(56) Entgegenhaltungen:
- EP-A1- 2 918 630
- EP-A2- 1 809 256
- DE-A1-102012 102 165
- DE-T2- 69 908 552
- Attain Group Ltd: "Bio-Glitter - Home", , 9. Januar 2018 (2018-01-09), XP055599640, Gefunden im Internet: URL:https://web.archive.org/web/2018010903 4240/https://www.bio-glitter.com/ [gefunden am 2019-06-26]

## Beschreibung

Die vorliegende Erfindung betrifft Glitter, umfassend eine Folie, die Celluloseacetat umfasst, wobei die Folie wenigstens 60 Gew.% Celluloseacetat umfasst, bezogen auf das Gewicht der Folie, wobei der Glitter ferner mit einem Metall beschichtet ist, dessen Verwendung in kosmetischen Produkten, sowie kosmetische Produkte, welche den Glitter umfassen.

### Stand der Technik

Glitter finden vielfach Anwendung zur Erzeugung eines glitzernden Oberflächeneffektes und werden unter anderem und insbesondere in kosmetischen Artikeln eingesetzt. Zur Herstellung derartiger Glitter werden Folien oder Filme aus Kunststoff verwendet, die mittels eines Schneidvorganges in einzelne, vergleichbar kleine Partikel geschnitten werden. Hauptsächlich werden derzeit Filme aus Polyethylenterephthalat für die Herstellung von Glitter verwendet.

Ein beispielhaftes Verfahren zur Herstellung solcher Glitter ist in der DE 102010001971 A1 offenbart. Hierin werden Glitter offenbart, die von allen Seiten beschichtet werden. Weitere Glitter werden auch offenbart in DE 699 08 552 T2, DE 10 2012 102165 A1, EP 1 809 256 A2, EP 2 918 630 A1 und insbsondere "Attain Group Ltd: "Bio-Glitter - Home", 9. Januar 2018 (2018-01-09), siehe :URL:https://web.archive.org/web/20180109034240/https://www.bio-glitter.com/.

Es besteht jedoch weiterhin ein Bedarf an Glitter, welche verbesserte Glanzeffekte aufweisen. Weiterhin ist es wünschenswert, Glitter mit einem verbesserten Skin-Feeling bereitzustellen, also solche, die bei einer Anwendung in kosmetischen Produkten ein weicheres und angenehmeres Gefühl auf der Haut hervorrufen. Darüber hinaus besteht ein Bedarf an Glitter, welche eine bessere Flächenabdeckung (Deckkraft) aufweisen.

Der Erfindung liegt daher die Aufgabe zugrunde, Glitter bereitzustellen, welche verbesserte Glanzeffekte und ein verbessertes Skin-Feeling aufweisen. Weiterhin ist es Aufgabe der Erfindung, Glitter bereitzustellen, welche eine bessere Flächenabdeckung (Deckkraft) aufweisen und welche aufgrund Ihrer guten Verträglichkeit in Kosmetikprodukten eingesetzt werden können.

### Zusammenfassung der Erfindung.

Bisher wurde für die Herstellung von Glitter hauptsächlich Folien auf Basis von Polyethylenterephthalat verwendet. Es hat sich überraschend gezeigt, dass die Aufgabe der Erfindung, also Glitter mit verbessertem Glanzeffekt und verbessertem Skin-Feeling, sowie Glitter mit verbesserter Flächenabdeckung (Deckkraft) bereitzustellen, gelöst werden kann, indem bei der Herstellung von Glitter Folien auf Basis von Celluloseacetat, wie in den Ansprüche beschrieben, verwendet werden.

Die vorliegende Erfindung betrifft somit Glitter umfassend eine Folie auf Basis von Celluloseacetat. Weiterhin betrifft die Erfindung die Verwendung von Glitter in kosmetischen Formulierungen.

In einem ersten Aspekt ist die vorliegende Erfindung auf Glitter, umfassend eine Folie, die Celluloseacetat umfasst, strikt wie in Anspruch 1 definiert, gerichtet.

Weiterhin offenbart sind die Verwendung der erfindungsgemäßen in kosmetischen Produkten sowie kosmetische Produkte, umfassend die erfindungsgemäßen Glitter. Weitere bevorzugte Ausgestaltungen der vorliegenden Erfindung ergeben sich aus den abhängigen Ansprüchen und der folgenden detaillierten Beschreibung der Erfindung.

### Beschreibung der Figuren

Die beiliegenden Zeichnungen sollen Ausführungsformen der vorliegenden Erfindung veranschaulichen und ein weiteres Verständnis dieser vermitteln. Im Zusammenhang mit der Beschreibung dienen sie der Erklärung von Konzepten und Prinzipien der Erfindung. Andere Ausführungsformen und viele der genannten Vorteile ergeben sich im Hinblick auf die Zeichnungen. Die Elemente der Zeichnungen sind nicht notwendigerweise maßstabsgetreu zueinander dargestellt. Gleiche, funktionsgleiche und gleich wirkende Elemente, Merkmale und Komponenten sind in den Figuren der Zeichnungen, sofern nichts anderes ausgeführt ist, jeweils mit denselben Bezugszeichen versehen.

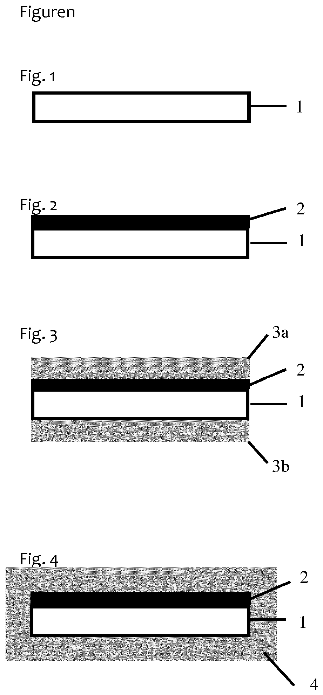

Figuren 1 bis 4 zeigen schematisch erfindungsgemäße Glitter.

### Detaillierte Beschreibung der Erfindung

### Definitionen

So nicht anderweitig definiert haben hierin verwendete technische und wissenschaftliche Ausdrücke dieselbe Bedeutung, wie sie von einem Fachmann auf dem Fachgebiet der Erfindung gemeinhin verstanden wird.

Mengenangaben im Rahmen der vorliegenden Erfindung beziehen sich auf Gew.%, soweit nicht anderweitig angegeben oder aus dem Kontext ersichtlich ist. In der Folie, die Celluloseacetat umfasst, ergänzen sich die Gew.%-Anteile auf 100 Gew.%.

Glitter sind kleine Partikel mit verschiedensten Formen. Insbesondere weisen sie eine Größe, beispielsweise einen maximalen Durchmesser in einer Hauptausdehnungsrichtung des Partikels, von 0,02 mm bis 7,0 mm, bevorzugt 0,050 mm bis 6,0 mm, beispielsweise 0,06 mm bis 2,0 mm, z.B. 0,1 mm bis- 0,5 mm auf, beispielsweise von 100 µm bis 200 µm, auf. Hinsichtlich der Form sind die Glitterpartikel nicht besonders beschränkt und können beispielsweise als Plättchen, Nadeln, Quader, etc. vorliegen, oder zu bestimmten Formen gestanzt sein, beispielsweise Sechsecken, Quadraten, Kreisen, Ovalen, Sternen, etc. Gemäß bestimmten Ausführungsformen sind die Glitter flach ausgeführt, beispielsweise als Plättchen mit verschiedensten Formen, z.B. auch sechseckig, rechteckig, quadratisch, sternförmig, rund, oval, etc., wobei bevorzugt die Dicke der Plättchen zwischen 4 µm und 50 µm, beispielsweise zwischen 5 µm und 45 µm, z.B. zwischen 10 µm und 35 µm, beispielshaft zwischen 14 µm und 23 µm liegen kann, und/oder die Größe, beispielsweise ein maximaler Durchmesser in einer Hauptausdehnungsrichtung der Glitter, von 0,02 mm bis 7,0 mm, bevorzugt 0,050 mm bis 6,0 mm, beispielsweise 0,06 mm bis 2,0 mm, z.B. 0,1 mm bis- 0,5 mm betragen kann.

In einem ersten Aspekt betrifft die vorliegende Erfindung Glitter, umfassend eine Folie, die Celluloseacetat umfasst. Hierbei ist das Celluloseacetat nicht besonders beschränkt, beispielsweise hinsichtlich des Substitutionsgrads.

Gemäß bestimmten Ausführungsformen umfasst die Folie wenigstens 60 Gew.% Celluloseacetat, bevorzugt mehr als 60 Gew.%, weiter bevorzugt mehr als 70 Gew.%, noch weiter bevorzugt mehr als 80 Gew.%, und insbesondere bevorzugt mehr als 90 Gew.%, beispielsweise mehr als 95 Gew.% oder sogar mehr als 99 Gew.%, bezogen auf das Gewicht der Folie.

Daneben können jedoch auch weitere Zusatzstoffe und/oder ein oder mehr weitere Polymere wie beispielsweise Cellulose, modifizierte Cellulose, regenerierte Cellulose, Stärke, Polymilchsäure (PLA), Polybutylenadipat-terephthalat (PBAT), Polybutylensuccinat (PBS), thermoplastische Stärke (TPS), Polyhydroxyalkanoat (PHA), etc., in einer Menge von bis zu 40 Gew.%, bevorzugt weniger als 40 Gew.%, weiter bevorzugt weniger als 30 Gew.%, noch weiter bevorzugt weniger als 20 Gew.%, und insbesondere bevorzugt weniger als 10 Gew.%, beispielsweise weniger als 5 Gew.%, oder sogar weniger als 1 Gew.%, in der Folie vorhanden sein, welche nicht besonders beschränkt sind.

Beispielsweise kann die Folie als Zusatzstoff gemäß bestimmten Ausführungsformen Glyceroltriacetat/Glycerintriacetat (Triacetin) enthalten. Der Massenanteil von Glyceroltriacetat kann hierbei bevorzugt weniger als 40 Gew.%, weiter bevorzugt weniger als 30 Gew.%, noch weiter bevorzugt weniger als 20 Gew.%, und insbesondere bevorzugt weniger als 10 Gew.%, beispielsweise weniger als 5 Gew.%, oder sogar weniger als 1 Gew.%, betragen, bezogen auf das Gewicht der Folie. Auch kann die Folie kein Glyceroltriacetat enthalten. Alternativ oder zusätzlich kann die Folie gemäß bestimmten Ausführungsformen Additive enthalten, welche z.B. als Antiblock- und/oder Gleithilfsmittel wirken können. Diese Antiblock und/oder Gleithilfsmittel sind nicht besonders beschränkt und können beispielsweise amorphe Kieselsäuren, Fettsäureamide und/oder Talk umfassen. Die Zugabemenge für solche Antiblock und/oder Gleithilfsmittel kann gemäß bestimmten Ausführungsformen o - 2,5 Gew.%, bevorzugt 0 - 1,5 Gew.%, weiter bevorzugt 0 - 1 Gew.% betragen, bezogen auf das Gewicht der Folie.

Gemäß bestimmten Ausführungsformen besteht die Folie im Wesentlichen aus Celluloseacetat oder besteht sogar aus Celluloseacetat, abgesehen von unvermeidbaren Verunreinigungen.

Gemäß bestimmten Ausführungsformen ist der Glitter ferner mit einem Metall, vorzugsweise Aluminium, Silber, Gold und/oder Kupfer, bevorzugt Aluminium, beschichtet. Die Beschichtung kann hierbei gemäß bestimmten Ausführungsformen auf einer Seite, auf zwei gegenüberliegenden Seiten, auf der gesamten Folie oder in anderer Weise erfolgen.

Gemäß bestimmten Ausführungsformen ist die Folie oder eine Metallschicht, beispielsweise eine Aluminiumschicht, mit einer Beschichtung auf Basis von Cellulose oder modifizierter Cellulose, bevorzugt auf Cellulosenitrat-Basis, Celluloseacetatbutyrat-Basis, Celluloseacetatpropionat-Basis und/oder Celluloseacetat-Basis, und/oder auf Polyurethan-, Acrylat-, Styrol-Acrylat- und/oder Epoxidbasis beschichtet. Auch hier kann die Beschichtung auf einer Seite, zwei gegenüberliegenden Seiten, auf der gesamten Metallschicht, beispielsweise Aluminiumschicht, der Folie und der Metallschicht, beispielsweise Aluminiumschicht, oder auf andere Weise vorhanden sein.

Insbesondere bevorzugt sind Beschichtungen auf Basis von Cellulose oder modifizierter Cellulose, bevorzugt auf Cellulosenitrat-Basis, Celluloseacetatbutyrat-Basis, Celluloseacetatpropionat-Basis und/oder Celluloseacetat-Basis, insbesondere Celluloseacetat-Basis.

Darüber hinaus kann der erfindungsgemäße Glitter auch weitere farb- und/oder effektgebende Schichten umfassen, wie sie dem Fachmann bekannt sind und die ein Fachmann geeignet beschichten kann, beispielsweise aus der Gasphase oder aus Flüssigkeit/Lösung.

Gemäß bestimmten Ausführungsformen weist die Folie der erfindungsgemäßen Glitter eine Dicke von 5 µm oder mehr, bevorzugt von mehr als 10 µm, weiter bevorzugt von mehr als 12 µm, und/oder eine Dicke von 40 µm oder weniger, beispielsweise weniger als 40 µm, bevorzugt weniger als 30 µm, weiter bevorzugt weniger als 28 µm und besonders bevorzugt weniger als 25 µm, beispielsweise auch weniger als 20 µm oder sogar weniger als 15 µm, auf.

Insbesondere bei diesen dicken Folien umfassend Celluloseacetat kann im erfindungsgemäßen Glitter eine weiter verbesserte gleichmäßige Verteilung in der kosmetischen Formulierung erreicht werden. Nach der Applikation auf der Haut ergeben sich dadurch weiter verbesserte Glanzeffekte und eine weiter verbesserte Flächendeckung (Deckkraft).

Gemäß bestimmten Ausführungsformen kann die Folie zudem transparent oder im Wesentlichen transparent, beispielsweise mit einer Transmissivität gegenüber Licht im sichtbaren Bereich von 380 bis 780 nm, von 70%, oder 80%, oder 90% oder mehr, sein. Gemäß bestimmten Ausführungsformen weist die Folie also eine Transmissivität gegenüber Licht im Wellenlängenbereich von 380 bis 780 nm von mindestens 70%, bevorzugt mindestens 80%, weiter bevorzugt mindestens 90%, auf.

Die Folie kann gemäß weiteren Ausführungsformen gefärbt oder ungefärbt sein, ist gemäß bestimmten Ausführungsformen jedoch ungefärbt. Zudem kann in bestimmten Ausführungsformen auf die Beschichtung mit Metall, bevorzugt Aluminium, oder die Folie und die Metallbeschichtung eine farbgebende Schicht aufgebracht werden.

Gemäß bestimmten Ausführungsformen weist die Folie ein E-Modul von weniger als 3000 Nmm⁻² auf, bevorzugt 2000 - 2500 Nmm⁻², gemessen gemäß ASTM D883.

Der vergleichsweise geringe E-Modul - entspricht einer geringeren Steifigkeit - wirkt sich positiv auf das weiche und angenehme Hautgefühl aus. Somit ist auch ein Glitter offenbart umfassend eine Folie, welche ein E-Modul von weniger als 3000 Nmm⁻², bevorzugt 2000 - 2500 Nmm⁻², gemessen gemäß ASTM D883, aufweist. Darüber hinaus ist die Folie für diesen weiteren, zweiten Aspekt der Erfindung nicht beschränkt und kann aus einem beliebigen Material sein, beispielsweise Cellulosederivate wie Celluloseacetat, Polyethylen (PE), Polypropylen (PP), PET, Cellulose, regenerierte Cellulose, Stärke, PLA, und/oder weitere polymere Materialien und/oder Mischungen davon umfassen oder daraus bestehen. Auch dieser Glitter des zweiten Aspekts kann weiterhin die oben genannten und nachfolgend angegebenen beispielhaften Ausgestaltungen aufweisen, wobei hier das Material der Folie nicht auf eines umfassend Celluloseacetat beschränkt ist. Ebenfalls offenbart sind natürlich auch ein kosmetisches Produkt umfassend einen solchen Glitter des zweiten Aspekts, welches nicht besonders beschränkt ist und wie nachfolgend angegeben ausgestaltet sein kann, wie auch die Verwendung des Glitters des zweiten Aspekts in einem kosmetischen Produkt.

Insgesamt kann aufgrund der verbesserten mechanischen Eigenschaften (E-Modul, Gleitfähigkeit) der im erfindungsgemäßen Glitter des ersten Aspekts verwendeten Folie ein deutlich verbessertes Skin-Feeling mit den erfindungsgemäßen Glitter des ersten Aspekts, beispielsweise in einem kosmetischen Produkt, erreicht werden.

Gemäß bestimmten Ausführungsformen kann die Folie in einem erfindungsgemäßen Glitter auch mit einer Hologramm-Prägung hergestellt werden, wie sie bei herkömmlichen Glitter bekannt ist, beispielsweise aus der US 5,810,957 oder EP2 163 381 A bekannt ist, wobei auf die beiden Dokumente hinsichtlich der Holgramm-Prägung Bezug genommen wird. Überraschenderweise lässt sich die Holgramm-Prägung auch auf der Folie umfassend Celluloseacetat realisieren. Zur Herstellung können hierbei gängige Verfahren verwendet werden, wie das sogenannte Soft-Embossing und das Hard-Embossing.

Nachfolgend wird die Erfindung näher erläutert anhand beispielhafter Ausführungsformen erfindungsgemäßer Glitter, insbesondere des ersten Aspekts, welche in Figuren 1 bis 4 gezeigt sind. Die Figuren zeigen hierbei schematisch Schnittansichten durch Glitter mit einem erfindungsgemäßen Aufbau.

Erfindungsgemäße Glitter umfassen hierbei beispielsweise Glitterpartikel mit einer Größe von 100 µm bis 200 µm und einer Dicke von 14 µm bis 23 µm, sind jedoch nicht hierauf beschränkt. Gemäß einer ersten beispielhaften Ausführungsform, die in Fig. 1 dargestellt ist, können transparente, im Wesentlichen als aus Celluloseacetat bestehenden Partikel 1 als Folie der erfindungsgemäßen Glitter durch Schneiden einer Celluloseacetat-Folie gewonnen werden.

Gemäß einer weiteren Ausführungsform, die in Fig. 2 dargestellt ist können die im Wesentlichen aus Celluloseacetat bestehenden Partikel 1 einseitig mit einer Metallschicht, beispielsweise einer Aluminiumschicht 2, beschichtet sein. Eine Beschichtung aus Aluminium kann hierbei vorzugsweise unter Vakuum aufgedampft werden.

Darüber hinaus können die einseitig mit Aluminium 2 beschichteten, im Wesentlichen aus Celluloseacetat bestehenden Partikel 1 in einer weiteren Ausführungsform, wie in Fig. 3 gezeigt, zusätzlich auf zwei gegenüberliegenden Seiten gleichmäßig mit Schichten 3a, 3b aus beispielsweise Celluloseacetat beschichtet sein.

Alternativ können die einseitig mit Aluminium 2 beschichteten, im Wesentlichen aus Celluloseacetat bestehenden Partikel 1 in einer weiteren Ausführungsform, wie in Fig. 4 gezeigt, auf allen Seiten gleichmäßig mit einer Schicht 4 aus beispielsweise Celluloseacetat beschichtet sein.

In den Figuren 1 bis 4 kann die im Wesentlichen aus Celluloseacetat bestehende Folie gemäß alternativen Ausgestaltungen auch gefärbt sein. In den Figuren 3 bis 4 können zudem die Schichten aus Celluloseacetat 3a, 3b und/oder 4 auf den im Wesentlichen aus Celluloseacetat bestehenden Partikeln gemäß alternativen Ausgestaltungen auch gefärbt sein.

Das Verfahren zur Herstellung der erfindungsgemäßen Glitter ist nicht besonders beschränkt. Eine Folie, insbesondere umfassend Celluloseacetat, kann hierbei geeignet zu Partikeln geeigneter Größe geschnitten und ggf. auf übliche Weise mit Metall, Polymer, etc. beschichtet werden. Hierbei können sich eine geringe Zugfestigkeit und geringe Bruchdehnung, beispielsweise insbesondere von Celluloseacetat-Folien, auf die Verarbeitbarkeit bei der Glitterherstellung auswirken. Bei der Glitterherstellung werden die Folien typischerweise "von Rolle" verarbeitet. Dabei wirken in den Verarbeitungsmaschinen (z.B. Folienbeschichtung und/oder Glitterschneidmaschine) üblicherweise große Zugkräfte auf die Folien. Bei der Verarbeitung ist es daher erforderlich, die auftretenden Zugkräfte so gering wie möglich zu halten, um ein Abreißen der Folie in den Verarbeitungsmaschinen zu vermeiden. Dies kann beispielsweise durch eine entsprechend gestaltete Bahnführung in den Verarbeitungsmaschinen und die entsprechende Auslegung der bahnführenden Transportwalzen erzielt werden, um zu große Zugkräfte auf die Folie zu vermeiden.

Das Aufbringen verschiedener Beschichtungen ist nicht besonders beschränkt und kann beispielsweise aus der Gasphase und/oder aus Lösung erfolgen.

Ein weiterer Aspekt der vorliegenden Erfindung ist auf die Verwendung von erfindungsgemäßen Glittern in kosmetischen Produkten gerichtet. Das kosmetische Produkt ist hierbei nicht besonders beschränkt. Kosmetische Produkte umfassen hierbei beispielsweise Pasten, Salben, Cremes, Emulsionen, Lösungen, Lippenstift, Lip Gloss, Mascara, Mousse, Eye Shadow, Eye Liner, Puder, gepresste Puder, loses Glitterpulver, Nagellack, Seifen, Shampoo, Sonnenschutzmittel, Lotionen, Aerosol-Sprays, usw., welche die Glitter in üblichen Mengen in den Formulierungen enthalten können.

Zudem offenbart ist ein kosmetisches Produkt, welches erfindungsgemäße Glitter umfasst. Das kosmetische Produkt ist hierbei nicht besonders beschränkt, und es kann sich beispielsweise um eine Paste, eine Salbe, eine Creme, eine Emulsion, eine Lösung, einen Lippenstift, Lip Gloss, Mascara, Mousse, Eye Shadow, Eye Liner, Puder, gepressten Puder, loses Glitterpulver, Nagellack, Seife, Shampoo, Sonnenschutzmittel, eine Lotion, ein Aerosol-Spray, usw, handeln, wobei die Glitter in üblichen Mengen im kosmetischen Produkt enthalten sein können, beispielsweise zwischen 0,01 und 75 Gew.%, z.B. zwischen 1 und 10 Gew.% bezogen auf das kosmetische Produkt, oder sogar bis zu 100 Gew.% im Falle von Puder und losem Glitterpulver. Daneben können in den kosmetischen Produkten die üblichen Bestandteile vorhanden sein, wie Trägerstoffe, Füllstoffe, Öle, Wachse, Fette, Emulgatoren, Antioxidationen, Filmbildner, Geruchs- und/oder Geschmacksstoffe, Stabilisatoren, Lösemittel, Tenside, Konservierungsmittel, Verdicker, rheologische Additive, Farbstoffe, Vitamine, Puffersubstanzen, kosmetische Wirkstoffe, hautaktive Stoffe, z.B. hautpflegenden Stoffe, UV-Filter, etc., welche alle nicht besonders beschränkt sind. Die kosmetischen Produkte können beispielsweise hydrophiler, hydrophober und/oder lipophiler Natur sein. Entsprechende Bestandteile sind beispielsweise aus der DE 102005055576 A1 bekannt, auf die beispielhaft in Bezug auf kosmetische Formulierungen zur Herstellung kosmetischer Produkte Bezug genommen wird.

Die obigen Ausführungsformen, Ausgestaltungen und Weiterbildungen lassen sich, sofern sinnvoll, beliebig miteinander kombinieren. Weitere mögliche Ausgestaltungen, Weiterbildungen und Implementierungen der Erfindung umfassen auch nicht explizit genannte Kombinationen von zuvor oder im Folgenden bezüglich der Ausführungsbeispiele beschriebenen Merkmalen der Erfindung. Insbesondere wird der Fachmann auch Einzelaspekte als Verbesserungen oder Ergänzungen zu der jeweiligen Grundform der vorliegenden Erfindung hinzufügen.

Die Erfindung wird im Anschluss mit Bezug auf verschiedene Beispiele davon weiter im Detail erläutert. Die Erfindung ist jedoch nicht auf diese Beispiele beschränkt.

### Beispiele

### Beispiel 1: Glitter

Erfindungsgemäße Glitter gemäß Figur 2 mit einer Größe von 100 µm bis 200 µm und einer Dicke von 14 µm bis 23 µm einer Celluloseacetat-Folie in den Glittern wurden durch Beschichten einer Celluloseacetat-Folie mit Aluminium durch Aufdampfen auf einer Seite und Schneiden auf die vorgegebene Größe hergestellt.

Zum Vergleich wurden Glitter mit Folien aus Polymichsäure (PLA, polylactic acid), regenerierter Cellulose und Polyethylenterephthalat (PET) durch entsprechende Verfahren mit dergleichen Größe und Beschichtung hergestellt.

Für die verschiedenen Materialien ergeben sich hierbei unterschiedliche E-Module, welche in Tabelle 1 angegeben sind.

**Tabelle 1: E-Module der Folien in den Glittern in Beispiel 1**

| | **Celluloseacetat** | **PLA** | **Regenerierte Cellulose** | **PET** |
|---|---|---|---|---|
| E-Modul [Nmm⁻²] ASTM D883 | 2000 - 2500 | 3500 - 4000 | 3000 - 3500 | 3600 - 4500 |

Marktüblich sind neben den herkömmlichen PET (Polyethylenterephthalat)-basierten Glittern auch Glitter erhältlich, die auf biologisch abbaubaren Folien basieren. Entsprechende Glitter sind hergestellt aus PLA (Polylactose) oder regenerierter Cellulose bekannt und kommerziell erhältlich. CA weist hierbei überraschenderweise im Vergleich zu PLA eine deutlich bessere Abbaubarkeit auf.

Im Vergleich zu PLA und regenerierter Cellulose ermöglicht die vergleichsweise hohe Temperaturbeständigkeit bei Celluloseacetat (CA) daneben die Verwendung in kosmetischen Produkten wie Lippenstift-Formulierungen, deren Herstellung typischerweise bei Temperaturen von bis zu 100 °C erfolgt.

Im Vergleich zu PLA und regenerierter Cellulose weisen Filme auf Basis von CA zudem eine bessere chemische Beständigkeit auf, beispielsweise getestet als Beständigkeit gegen Ethanol. Das gute Beständigkeitsprofil von CA ermöglicht die Verwendung der erfindungsgemäßen Glitter in den meisten kosmetischen Formulierungen.

Weitere kommerziell verfügbare, biologisch abbaubare Folien bestehen aus Polybutylenadipat-terephthalat (PBAT), Polybutylensuccinat (PBS), thermoplastischer Stärke (TPS) oder Polyhydroxyalkanoat (PHA). Aufgrund materialtechnischer Eigenschaften sind diese Folien jedoch nicht für die Glitterherstellung geeignet. PBAT, PBS und TPS bilden weiche, elastische Filme, die sich nicht zu Glitterpartikeln schneiden lassen - dies hängt mit den niedrigen Glasübergangstemperaturen (Tg) dieser Polymere zusammen. PBS hat eine Glasübergangstemperatur von - 30 °C. Zum Vergleich liegt die Glasübergangstemperatur für PLA bei + 50 °C. TPS ist darüber hinaus nicht hydrolysebeständig. Auf PHA basierenden Filme sind weißlich trübe und ergeben im Vergleich zu transparenten Filmen keine hochgläzende, reflekierende Oberflächen.

Somit zeigt sich die hervorragende Verwendbarkeit von Celluloseacetat-Folien in erfindungsgemäßen Glittern im Vergleich zu denen des Stands der Technik.

### Beispiel 2: Applikationsbeispiele in kosmetischen Produkten

In den nachfolgenden Formulierungsbeispielen werden jeweils ein erfindungsgemäßer Glitter (Glitterbeispiel gemäß Fig. 2 aus Beispiel 1) und ein dem Stand der Technik entsprechender Glitter (Vergleichsglitter bestehend aus einem Polyethylenterephthalat-Film mit Al-Beschichtung mit gleicher Größe aus Beispiel 1) vergleichend beurteilt.

Mit diesen Glittern wurden verschiedene kosmetische Produkte auf übliche Weise hergestellt, mit den Bestandteilen/Inhaltsstoffen und Anteilen (Gew.%, bezogen auf das Produkt) gemäß den jeweiligen Angaben in Tabellen 2, 3, 4 und 5.

### Produkt 1: Gesichtspuder

**Tabelle 2: Zusammensetzung des Gesichtspuders**

| | **Inhaltsstoff** | **%W/W** |
|---|---|---|
| **Phase A** | Talk | 19,5 |
| | Glimmer (Mica) | 39,3 |
| | Nylon 12 | 8,0 |
| | Siliziumdioxid (Silica) | 5,0 |
| | Zinkstearat | 6,0 |
| | Propylparaben | 0,2 |
| | CI 77491 (Eisenoxid rot; Iron Oxide Red) | 0,3 |
| | CI 77492 (Eisenoxid gelb; Iron Oxide Yellow) | 0,7 |
| **Phase B** | Tridecylstearat (und) Tridecyltrimellitat (und) Dipentaerythrityl Hexacaprylate/Hexacarate | 2,0 |
| **Phase C** | Glitter | 19,0 |
| | **Summe** | 100,0 |

### Herstellung:

1. Inhaltsstoffe der Phase A mischen
2. Phase B und Phase C in die Phase A mischen Der Gesichtspuder mit dem erfindungsgemäßen Glitter ist glänzender und weist ein weicheres Hautgefühl auf als der Gesichtspuder mit dem Vergleichsglitter.

### Produkt 2: Lidschatten (gepresst)

**Tabelle 3: Zusammensetzung des Lidschattens**

| | **Inhaltsstoff** | **%W/W** |
|---|---|---|
| **Phase A** | Talk | 32,9 |
| | Glimmer (Mica) | 21,9 |
| | Bornitrid | 2,0 |
| | Siliziumdioxid (Silica) | 5,0 |
| | Magnesiumstearat | 3,0 |
| | Propylparaben | 0,2 |
| **Phase B** | Dimethicone | 5,0 |
| | METHOXY PEG-17/METHOXY PEG-11/HDI CROSSPOLYMER | 5,0 |
| | Glitter | 25,0 |
| | **Summe** | 100,0 |

### Herstellung:

1. Inhaltsstoffe der Phase A mischen
2. Inhaltsstoffe der Phase B mischen
3. Phase A und Phase B homogen vermischen
4. 30 min bei 150 bar pressen

Der Lidschatten mit dem erfindungsgemäßen Glitter ist glänzender und weist eine bessere Deckkraft auf als der Lidschatten mit dem Vergleichsglitter.

### Produkt 3: Lipgloss

**Tabelle 4: Zusammensetzung des Lipgloss**

| | **Inhaltsstoff** | **%W/W** |
|---|---|---|
| **Phase A** | Paraffinum Liquidum | 42,7 |
| | Polyisobuten | 37,4 |
| | Triisodecyltrimellitat | 4,0 |
| | Mineralöl (und) Ethylen/Propylen/StyrolCopolymer & Butylen/Ethylen/StyrolCopolymer | 3,0 |
| | Silica Dimethylsilylat (Silica Dimethyl Silylate) | 2,0 |
| | Ethylhexylpalmitat | 2,0 |
| | Hydriertes Polyisobuten | 2,0 |
| | Polyethylen | 1,5 |
| | Sorbitansesquiisostearat | 1,0 |
| | Mineralwachs (Cera Microcristallina) | 0,5 |
| | Propylparaben | 0,2 |
| **Phase B** | Glitter | 3,7 |
| | **Summe** | 100,0 |

### Herstellung:

1. Inhaltsstoffe der Phase A homogen mischen
2. Phase B in Phase A einrühren

Der Lipgloss mit dem erfindungsgemäßen Glitter weist ein weicheres Hautgefühl auf als der Lipgloss mit dem Vergleichsglitter.

### Produkt 4: Eyeliner

**Tabelle 5: Zusammensetzung des Eyeliners**

| | **Inhaltsstoff** | **%W/W** |
|---|---|---|
| **Phase A** | Wasser | 73,5 |
| | Xanthan (Xanthan Gum) | 0,9 |
| | Glitter | 2,5 |
| **Phase B** | Propylenglycol | 2,0 |
| | Phenoxyethanol (und) Ethylhexylglycerin | 0,8 |
| | Polyurethan-35 | 19,5 |
| | Laureth-4 | 0,8 |
| | **Summe** | 100,0 |

### Herstellung:

1. Inhaltsstoffe der Phase A homogen mischen
2. Inhaltsstoffe der Phase B der Reihe nach zugeben und einmischen

Der Eyeliner mit dem erfindungsgemäßen Glitter weist eine gleichmäßigere Verteilung der Glitterpartikel auf und zeigt dadurch einen besseren Glanzeffekt als der Eyeliner mit dem Vergleichsglitter.

## Patentansprüche

1. Glitter, umfassend eine Folie, die Celluloseacetat umfasst, wobei die Folie wenigstens 60 Gew.% Celluloseacetat umfasst, bezogen auf das Gewicht der Folie, wobei der Glitter ferner mit einem Metall beschichtet ist.

2. Glitter nach Anspruch 1, wobei die Folie im Wesentlichen aus Celluloseacetat besteht.

3. Glitter gemäß einem der vorigen Ansprüche, wobei der Glitter mit Aluminium beschichtet ist.

4. Glitter gemäß einem der vorigen Ansprüche, wobei die Folie oder eine Metallschicht, beispielsweise eine Aluminiumschicht, mit einer Beschichtung auf Basis von Cellulose oder modifizierter Cellulose, bevorzugt auf Cellulosenitrat-Basis, Celluloseacetatbutyrat-Basis, Celluloseacetatpropionat-Basis und/oder Celluloseacetat-Basis, und/oder auf Polyurethan-, Acrylat-, Styrol-Acrylat- und/oder Epoxidbasis beschichtet ist.

5. Glitter gemäß einem der vorigen Ansprüche, wobei die Folie eine Dicke von 5 µm oder mehr aufweist, und/oder wobei die Folie eine Dicke von 40 µm oder weniger aufweist.

6. Glitter gemäß einem der vorigen Ansprüche, wobei die Folie eine Transmissivität gegenüber Licht im Wellenlängenbereich von 380 bis 780 nm von mindestens 70% aufweist.

7. Glitter gemäß einem der vorigen Ansprüche, wobei die Folie ein E-Modul von weniger als 3000 Nmm⁻² aufweist, bevorzugt 2000 - 2500 Nmm⁻², gemessen gemäß ASTM D883.

8. Verwendung von Glitter gemäß einem der Ansprüche 1 - 7 in kosmetischen Produkten.

9. Kosmetisches Produkt, umfassend einen Glitter gemäß einem der Ansprüche 1 - 7.

## Claims

1. Glitter comprising a film which comprises cellulose acetate, wherein the film comprises at least 60 wt% cellulose acetate based on the weight of the film, wherein the glitter is further coated with a metal.

2. Glitter according to claim 1, wherein the film essentially consists of cellulose acetate.

3. Glitter according to one of the preceding claims, wherein the glitter is coated with aluminium.

4. Glitter according to one of the preceding claims, wherein the film or a metal layer, for example an aluminium layer, is coated with a coating based on cellulose or modified cellulose, preferably based on cellulose nitrate, based on cellulose acetate butyrate, based on cellulose acetate propionate and/or based on cellulose acetate, and/or based on polyurethane, acrylate, styrene acrylate and/or epoxide.

5. Glitter according to one of the preceding claims, wherein the film has a thickness of 5 µm or more, and/or wherein the film has a thickness of 40 µm or less.

6. Glitter according to one of the preceding claims, wherein the film has a transmissivity for light in the wavelength range from 380 to 780 nm of at least 70%.

7. Glitter according to one of the preceding claims, wherein the film has an E modulus of less than 3000 Nmm⁻², preferably 2000 - 2500 Nmm⁻², measured according to ASTM D883.

8. Use of glitter according to one of claims 1-7 in cosmetic products.

9. Cosmetic product comprising a glitter according to one of claims 1-7.

## Revendications

1. Paillette, comprenant un film qui comprend de l'acétate de cellulose, dans laquelle le film comprend au moins 60 % en poids d'acétate de cellulose, par rapport au poids du film, la paillette étant en outre revêtue d'un métal.

2. Paillette selon la revendication 1, dans laquelle le film est essentiellement constitué d'acétate de cellulose.

3. Paillette selon l'une des revendications précédentes, la paillette étant revêtue avec de l'aluminium.

4. Paillette selon l'une des revendications précédentes, dans laquelle le film ou une couche métallique, par exemple, une couche d'aluminium, est revêtu(e) avec un revêtement à base de cellulose ou de cellulose modifiée, de préférence à base de nitrate de cellulose, à base de butyrate de cellulose, à base d'acéto-propionate de cellulose et/ou à base d'acétate de cellulose, et/ou à base de polyuréthane, d'acrylate, de styrène-acrylate et/ou d'époxyde.

5. Paillette selon l'une des revendications précédentes, dans laquelle le film présente une épaisseur de 5 µm ou plus, et/ou dans laquelle le film présente une épaisseur de 40 µm ou moins.

6. Paillette selon l'une des revendications précédentes, dans laquelle le film présente une transmissivité vis à vis de la lumière d'au moins 70 % dans la plage de longueurs d'onde de 380 à 780 nm.

7. Paillette selon l'une des revendications précédentes, dans laquelle le film présente un module élastique E inférieur à 3000 Nmm⁻², de préférence de 2000 à 2500 Nmm⁻², mesuré selon la norme ASTM D883.

8. Utilisation d'une paillette selon l'une des revendications 1 à 7 dans des produits cosmétiques.

9. Produit cosmétique, comprenant une paillette selon l'une des revendications 1 à 7.
